# EUROPEAN PATENT APPLICATION

(11) **EP 4 733 418 A1**
(43) Date of publication of application: **29.04.2026**
(21) Application number: 24208180.0
(22) Date of filing: 22.10.2024
(51) Int. Cl.: C12Q 1/689

(54) **METHOD FOR TAXONOMIC AND STRAIN TYPE IDENTIFICATION OF BACTERIA AND ARCHAEA**

(71) Applicant: Meola, Marco, 8910 Affoltern am Albis (CH)
(72) Inventor: Meola, Marco, 8910 Affoltern am Albis (CH)
(74) Representative: Braunpat AG

(57) **Abstract**

The present invention relates to a relates to a method for identifying microorganisms strain types using a single locus sequence typing technique in a sample. The present invention is furthermore related to a kit for use in said method.

## Description

### TECHNICAL FIELD

The present invention relates to a method for identifying microorganisms strain types using a single locus sequence typing technique in a sample. The present invention is furthermore related to a kit for use in said method.

### BACKGROUND OF THE INVENTION

The identification and differentiation of microbial species, particularly bacteria archaea and fungi, is essential in a wide range of fields including clinical and veterinary diagnostics, epidemiology, human and veterinary microbiomics and metagenomics, environmental monitoring, and food safety.

Traditional methods for microbial identification often rely on cultivation-based techniques, which can be time-consuming, labor-intensive, and may fail to identify non-culturable or slow-growing organisms.

Strain of species and subspecies resolution in bacterial diagnostics has become increasingly important in recent years, as it offers crucial insights for clinical decision-making, epidemiological investigations, and public health interventions. Traditional methods of bacterial identification often lack the precision needed to differentiate between closely related strains, which can have significant implications for patient care and outbreak management.

Bacterial species commonly possess multiple, non-identical copies of ribosomal RNA operons *(rrn)* distributed across their genomes. Despite the variability of single ribosomal genes within the ribosomal RNA operon, the 16S rRNA gene is used as a standard gene for phylogenetic analysis. Additionally, sequence variations are observed in the 23S and 5S rRNA genes and the internal transcribed spacer regions (ITS1 and ITS2) of the multiple ribosomal RNA operons within the genome.

The sequencing of specific gene targets, such as the 16S ribosomal RNA gene, has become a cornerstone for cultivation-free identification and classification of bacteria and archaea at species level. However, the limitations of the marker gene 16S rRNA gene in strain type resolution are primarily due to its highly conserved nature, which does not exhibit sufficient variability to differentiate between closely related strains. Additionally, the complexity of microbial populations, coupled with the presence of polymicrobial or contaminated samples, further complicates the ability to accurately resolve strains using existing sequencing methods.

The ability to accurately identify and characterize bacterial strains at high resolution is critical for several reasons: Strain-level identification can reveal important differences in virulence, antibiotic resistance profiles, and host interactions, allowing for more targeted and effective treatments. High-resolution typing enables better tracking of disease outbreaks, helping to identify sources of infection and transmission routes more accurately. Accurate strain identification can guide more appropriate antibiotic use, potentially slowing the development of resistance. In microbiome studies, strain-level resolution provides a more nuanced understanding of microbial community dynamics and host-microbe interactions and biomarker discovery of keystone strains.

There is therefore a need for improved methods that enhance the resolution of strain typing of microorganisms, such as bacteria, archaea, and fungi. These methods would provide more precise and accurate identification at the strain level, filling the gaps left by current approaches.

### SUMMARY OF THE INVENTION

The present invention provides a method for identifying strain types of bacterial species and/or subspecies in a sample the method comprising the steps of:
a) obtaining a sample containing one or more strain types of bacterial species and/or subspecies;
b) extracting DNA from said sample;
c) detecting from the extracted DNA the presence of at least one or more polynucleotides sequences which encode the full-length sequence of a bacterial ribosomal RNA operon encompassing a 16S rRNA gene and a 5S rRNA gene;
d) analyzing said at least one or more polynucleotides sequences as predictive of the identity of strain types of bacterial species and/or subspecies and comparing the at least one or more polynucleotides sequences with reference sequences of bacterial ribosomal RNA operon in a rrn-database; and
e) identifying the strain types of bacterial species and/or subspecies based on the at least one or more polynucleotides sequences which encode sequences of bacterial ribosomal RNA operons detected in the sample.

The present invention also provides a kit for use in said method comprising
a) at least one set of primers comprising forward primers targeting the 16S rRNA gene and reverse primers targeting the 5S rRNA gene wherein the sequence of the forward primer is SEQ ID NO: 3 and the sequence of the reverse primers is selected from SEQ ID NO: 1 and SEQ ID NO:2;
b) instructions for use to detect the presence in a sample of polynucleotides sequences which encode the full-length sequence of a bacterial ribosomal RNA operon encompassing a 16S rRNA gene and a 5S rRNA gene.

### DETAILED DESCRIPTION OF THE INVENTION

In one aspect, the present invention relates to a method for identifying microorganism strain in a sample, the method comprising the steps of:
a) obtaining a sample containing one or more microorganism strain types;
b) extracting DNA from said sample;
c) detecting from the extracted DNA the presence of at least one or more polynucleotides sequences which encode the full-length sequence of a microorganism ribosomal RNA operon;
d) analyzing said at least one or more polynucleotides sequences as predictive of the identity of the microorganism strain types and comparing the at least one or more polynucleotides sequences with reference sequences of microorganisms ribosomal RNA operon in a *rrn*-database; and
e) identifying the microorganism strain types based on the at least one or more polynucleotides sequences which encode sequences of microorganisms ribosomal RNA operons detected in the sample.

The following definitions are supplied to facilitate the understanding of the present invention.

As used herein, the term "comprise" is generally used in the sense of include, that is to say permitting the presence of one or more features or components.

As used herein, the singular form "a", "an" and "the" include plural references unless the context clearly dictates otherwise.

As used herein, a "sample" refers to a sample obtained from a subject or a sample taken from a natural environment, or a sample from a manufactured product.

As used herein, a "subject" is an animal, preferably, a mammal, more preferably, a human.

As used herein, an "polynucleotide" refers to a sequence of DNA residues that form a molecule. They can be found as single stranded DNA (ssDNA) molecule, or as double stranded DNA (dsDNA) molecule.

As used herein, an "oligonucleotide" refers to a sequence of DNA residues as single stranded DNA (ssDNA) molecule.

As used herein, a microorganism "strain" is a group of microorganisms that belong to the same species but share certain genetic characteristics not found in other members of the species.

As used herein, "microorganisms", such as bacteria, archaea, and fungi, have many strain types within a single species. Different strain types of an organism may have different biological characteristics, such as the ability to cause more severe diseases.

Taxonomy involves describing and defining living organisms in terms of species and organizing them into hierarchical categories called taxa. As used herein, "species" are the fundamental taxonomic units of biological classification. As used herein, "subspecies" is a rank below species.

As used herein, "ribosomal RNA operons" (*rrn*) in bacteria and archaea, typically codes for the 16S, 23S and 5S rRNAs, whereas "ribosomal RNA operons" in fungi codes for the 18S, 5.8S, and 28S rRNAs.

The full-length sequence of a microorganism ribosomal RNA operon comprises polynucleotides coding for rRNAs and other regions such as non-coding regions which are known as "internal transcribed spacer" (ITS). In addition to ITS, the full-length sequence may also comprise one or more tRNA genes depending on the microorganism strain types.

As used herein, the "full-length sequence" of a ribosomal RNA operon comprises at least 80%, 85%, 90%, 95%, 96%, 97%, 98% or at least 99% of the ribosomal RNA operon. For example, in the present invention, the full-length sequence of a ribosomal RNA operon may comprise the sequence resulting from a PCR reaction with a forward primer such as SEQ ID NO:3 complementary to the nucleotides sequence at the 3' end of the antisense (3' to 5') strand of the target nucleotides sequence of the 16S rRNA gene and a reverse primer such as SEQ ID NO: 1 or 2 that are complementary to the 3' end of the sense (5' to 3') strand of the target nucleotides sequence of the 5S rRNA gene.

Advantageously, the method may be configured to simultaneously detect a plurality of microorganisms such as bacteria, archaea and/or fungi in one sample.

In the present invention, the sample may be obtained from a subject and is selected from the group consisting of whole blood, serum, saliva, sputum, urine, cerebrospinal fluid, stool, amniotic fluid, tissue sample such as biopsy, synovial fluid, swab sample, or a sample containing liquid or solid colonies of bacteria.

Alternatively, the sample may be selected from the group consisting of food, food supplement, water, soil, air or filtered samples.

Preferably, the present invention relates to a method for identifying strain types of bacterial species and/or subspecies in a sample the method comprising the steps of:
a) obtaining a sample containing one or more strain types of bacterial species and/or subspecies;
b) extracting DNA from said sample;
c) detecting from the extracted DNA the presence of at least one or more polynucleotides sequences which encode the full-length sequence of a bacterial ribosomal RNA operon encompassing a 16S rRNA gene and a 5S rRNA gene;
d) analyzing said at least one or more polynucleotides sequences as predictive of the identity of strain types of bacterial species and/or subspecies and comparing the at least one or more polynucleotides sequences with reference sequences of bacterial ribosomal RNA operon in a *rrn*-database; and
e) identifying the strain types of bacterial species and/or subspecies based on the at least one or more polynucleotides sequences which encode sequences of bacterial ribosomal RNA operons detected in the sample.

Preferably, the sample containing one or more strain types of bacterial species and/or subspecies is obtained from a subject.

As used herein, a "strain type" of bacteria comprises genetic variants or subtypes of a species and/or subspecies of bacteria. One or more "sequence type" of a "strain type" can be determined by a Multi-Locus Sequence Typing (MLST) technique.

Advantageously, the method is a single locus sequence typing method for identifying strain types of species and/or subspecies of bacteria in a sample. The method is based on detecting polynucleotides sequences which encode the full-length sequence of a bacterial ribosomal RNA operon encompassing a 16S rRNA gene and a 5S rRNA gene having sufficient variability to differentiate specifically between closely related strain types of bacteria.

Typically, bacterial ribosomal RNA operons are approximately 4800bp to 7500bp of nucleotides in length, with minor variants of 1700-3500bp of nucleotides in length wherein the 23S rRNA gene is missing.

As used herein, the full-length sequence of a bacterial ribosomal RNA operon encompasses all sequences from 5' to 3' including the 16S rRNA gene and 5S rRNA gene.

Preferably, the bacterial ribosomal RNA operon encompasses from 5' to 3':
a) a 16S rRNA gene;
b) a non-coding regions ITS1;
c) one or more tRNA gene;
d) a 23S rRNA gene;
e) a non-coding regions ITS2;
f) one or more tRNA gene; and
g) a 5S rRNA gene,
or the bacterial ribosomal RNA operon encompasses from 5' to 3':
a) a 16S rRNA gene;
b) a non-coding regions ITS;
d) one or more tRNA gene; and
e) a 5S rRNA gene.

ITS1 sequences located between the 16S and 23S ribosomal subunit genes comprise (i) nucleotide sequences with the proviso that tRNA are excluded, or (ii) nucleotide sequences comprising one or more tRNA.

ITS2 sequence located between the 23S and 5S ribosomal subunit genes comprise (i) nucleotide sequences with the proviso that tRNA are excluded, or (ii) nucleotide sequences comprising one or more tRNA.

Advantageously, in the present invention, the method does not require the sample to be cultured.

Bacterial isolate may be cultivated under bacterial growth conditions in step a) in order to extract a sufficient amount of DNA.

In the present invention, the detecting step c) may be carried out by a polymerase chain reaction using a set of primers for amplifying the full-length sequence of a bacterial ribosomal RNA operon encompassing a 16S rRNA gene and a 5S rRNA gene.

A "primer" is a short oligonucleotide sequence with a specific sequence that binds to sequences in a single-stranded DNA molecule and provides a starting point for DNA synthesis by DNA polymerase in a polymerase chain reaction (PCR).

Alternatively, the detecting step c) may be carried out by digesting the non-target DNA region while preserving the target region comprising the full-length sequence of a bacterial ribosomal RNA operon encompassing a 16S rRNA gene and a 5S rRNA gene. In that case, primers will be used as delimiting sequences to detect which region not to be digested and to amplify.

Preferably, the detecting step c) is carried out by a polymerase chain reaction using a set of primers for amplifying the full-length sequence of a bacterial ribosomal RNA operon encompassing a 16S rRNA gene and a 5S rRNA gene.

In the present invention, one or more set of primers can be used. The at least one set of primers comprises forward primers targeting the 16S rRNA gene and reverse primers targeting the 5S rRNA gene.

Forward primers and reverse primers comprise between 15 and 25 nucleotides in length, preferably between 17 and 23 nucleotides in length, more preferably 20 nucleotides in length.

Forward primers are designed to target the 5' of region of the 16S rRNA gene. For example, the sequence of the forward primer is
5'-AGRRTTYGATYHTDGYTYAG-3'
wherein
R is A or C;
Y is C or T/U;
H is A or C or T/U; and
D is A or G or T/U.

Reverse primers are designed to target the variable downstream region of the 5S rRNA gene. For example, the sequence of the reverse primers is selected from the group comprising
5'- GTTCGGNATGGRWHSRGGYG-3' and/or 5'-GTTCGRNAWGGDDHSRSGYG-3' wherein
R is A or C;
N is A or C or G or T/U;
W is A or T/U;
D is A or G or T/U;
H is A or C or T/U;
S is C or G; and
Y is C or T/U.

Any variation of the forward and reverse primers can be used. The variant primer may comprise at least 1, 2, 3, 4, or 5 substitutions and/or deletions.

In one embodiment, the method may further comprise a step of preparing a DNA library comprising the at least one or more polynucleotides sequences detected after step c).

In another embodiment, the analyzing step d) is carried out by DNA sequencing analysis of said at least one or more polynucleotides sequences.

In the present invention, the sequencing of the at least one or more polynucleotides sequences amplified for example by PCR is carried out. Sequences of the said polynucleotides sequences obtained are compared with reference sequences of microorganism ribosomal RNA operon in a *rrn*-database. The presence of the strain type of bacterial species and/or subspecies in a sample is determined if the sequencing of a polynucleotide sequence obtained is identical to that of a reference sequence in said *rrn*-database so as to obtain an indication of the strain type.

A sample may contain multiple strain types of bacterial species and/or subspecies.

In step d), said at least one or more polynucleotides sequences form long reads by DNA sequencing analysis of approximately 1700-6000 nucleotides in length that cover the entire ribosomal RNA operon. The entire or full-length ribosomal RNA operon comprises at least 80%, 85%, 90%, 95%, 96%, 97%, 98% or at least 99% of the ribosomal RNA operon.

Advantageously, polymorphic sites are detected more reliably from long reads obtained by high throughput sequencing using long-read sequencing devices, enhancing the resolution of strain typing or sequence typing of bacteria.

In the present invention, the rm-database comprises reference sequences of bacterial ribosomal RNA operons and for each reference sequence, the taxonomic classification, the sequence type, and the serotype of the unique *rrn* copies per genome as an indication of the strain type of bacterial species and/or subspecies.

As used herein, a "serotype" represents a subgroup of microorganisms within a species that share a unique set of surface antigens, allowing for more precise classification and epidemiological tracking of pathogens.

The rm-database will be regularly updated with new additional *rrn* from complete bacterial or archaeal genomes. In addition, the rm-database will be updated with any other strain designation of the unique *rrn* copies per genome. In the rrn-database, one copy of redundant identical copies of the ribosomal operon from different strains or within the same genome will be maintained in the *rrn*-database with multiple annotations indicating the presence of said sequence copy in different strains or within the same genome.

In addition to the identification of strain type of bacterial species and/or subspecies in a sample, the method is reliable for identifying bacteria ecotypes, clonal complexes, serotypes, pathovars, phagovars, genomovars in a sample using the *rrn* database.

Preferably, the *rrn* database comprises reference sequences of bacterial ribosomal RNA operons and for each reference sequence, the taxonomic classification, the sequence type, the serotype of the unique *rrn* copies per genome, the bacterial ecotype, the clonal complex, the pathovar, the genomovar and the lineage, as an indication of the strain type of bacterial species and/or subspecies.

As used herein, "bacterial ecotypes" are evolutionary lineages that are irreversibly separate, each with its own evolutionary tendencies and historical fate. A species in the bacterial world may be understood as an evolutionary lineage bound together by ecotype-specific periodic selection.

As used herein, a "clonal complex" represents a group of genetically similar bacterial isolates, typically defined by MLST data, that are believed to share a recent common ancestor and are useful for understanding bacterial population structures and epidemiology.

As used herein, a "pathovar" classification is based solely on symptoms and pathogenicity characteristics, without considering genetic or physical descriptions of the bacteria.

As used herein, a "genomovar" refers to genomic variants or variations within a species or group of closely related organisms.

As used herein, a "sequence type" (ST) in clinical microbiology is a numerical designation assigned to a microbial isolate based on the specific sequences of multiple housekeeping genes, as determined by Multilocus Sequence Typing (MLST). This system provides a standardized method for characterizing and comparing microbial strains, which is essential for epidemiological surveillance, outbreak investigation, and understanding the genetic relationships among pathogens.

As used herein, a" lineage" can be understood as a group of reference genomes inferred as being related to each other based on one or more similarities in the reference genomes.

Thus, the method of the invention may be useful in the diagnosis of diseases in a subject characterized by the presence of the strain type of bacterial species and/or subspecies identified in the sample of said subject.

In one embodiment, the bacteria may be a single or multi-drug resistant strain of bacteria.

The method may further comprise a step of treating the subject with at least one antibacterial agent against the strain types of species and/or subspecies of bacteria identified after step e).

Antibacterial agents exhibit antibacterial activity by killing or reducing the metabolic activity of pathogenic bacteria.

Thus, the method may further comprise a step of treating the subject by administering to said subject a pharmaceutically effective amount of an antibacterial agent selected from the group comprising Amoxicillin, Ampicillin (Pivampicillin, Hetacillin, Bacampicillin, Metampicillin, Talampicillin), Epicillin, Carbenicillin (Carindacillin), Ticarcillin, Temocillin, Azlocillin, Piperacillin, Mezlocillin, Mecillinam (Pivmecillinam), Sulbenicillin, Benzylpenicillin (G), Clometocillin, Benzathine benzylpenicillin, Procaine benzylpenicillin, Azidocillin, Penamecillin, Phenoxymethylpenicillin (V), Propicillin, Benzathine phenoxymethylpenicillin, Pheneticillin, Cloxacillin (Dicloxacillin, Flucloxacillin), Oxacillin, Meticillin, Nafcillin, Faropenem, Biapenem, Doripenem, Ertapenem, Imipenem, Meropenem, Panipenem, Tomopenem, Razupenem, Cefazolin, Cefacetrile, Cefadroxil, Cefalexin, Cefaloglycin, Cefalonium, Cefaloridine, Cefalotin, Cefapirin, Cefatrizine, Cefazedone, Cefazaflur, Cefradine, Cefroxadine, Ceftezole, Cefaclor, Cefamandole, Cefminox, Cefonicid, Ceforanide, Cefotiam, Cefprozil, Cefbuperazone, Cefuroxime, Cefuzonam, Cefoxitin, Cefotetan, Cefmetazole, Loracarbef, Cefixime, Ceftazidime, Ceftriaxone, Cefcapene, Cefdaloxime, Cefdinir, Cefditoren, Cefetamet, Cefmenoxime, Cefodizime, Cefoperazone, Cefotaxime, Cefpimizole, Cefpiramide, Cefpodoxime, Cefsulodin, Cefteram, Ceftibuten, Ceftiolene, Ceftizoxime, Flomoxef, Latamoxef, Cefepime, Cefozopran, Cefpirome, Cefquinome, Ceftobiprole, Ceftaroline, CXA-101, RWJ-54428, MC-04,546, ME1036, BAL30072, SYN 2416, Ceftiofur, Cefquinome, Cefovecin, Aztreonam, Tigemonam, Carumonam, RWJ-442831, RWJ-333441, and/or RWJ-333442.

The method disclosed herein may be useful for the prevention, management or treatment of diseases or conditions caused by or related to bacterial infection in a subject.

The subject can be a human patient having a disease or health condition caused by or related to bacterial infection, including but not limited to nosocomial infection, otitis, conjunctivitis, pneumonia, bacteremia, sinusitis, pleural empyema and endocarditis, intravascular or endothelial infections, osteomyelitis, meningitis and chronic respiratory diseases such as asthma, chronic obstructive pulmonary disease (COPD), occupational lung diseases and pulmonary hypertension.

In another embodiment, the method disclosed herein may be applied for microbiota or microbiome analyses and useful for the prevention, management or treatment of diseases or health conditions caused by or related to the microbiome or microbiota in a subject. For example, the microbiota analysis may be carried out by amplification-based sequencing and the microbiome analysis may be carried out by shotgun metagenomic sequencing of all DNA.

The diseases or health conditions caused by or related to the microbiome or microbiota can be for example selected from the group comprising gastrointestinal disorders, metabolic disorders, autoimmune and inflammatory diseases, allergic and atopic conditions, neurological and psychiatric disorders, cardiovascular diseases, skin conditions, respiratory conditions, cancer, genitourinary and reproductive health, metabolic bone diseases, infectious diseases, mental health disorders, chronic kidney disease, non-alcoholic steatohepatitis (NASH), fibromyalgia, chronic fatigue syndrome and allergic diseases.

Gastrointestinal disorders are for example selected from inflammatory bowel disease (ibd), crohn's disease, ulcerative colitis, irritable bowel syndrome (ibs), celiac disease, colorectal cancer, clostridioides difficile infection, gastroesophageal reflux disease (gerd), peptic ulcer disease, small intestinal bacterial overgrowth (sibo) and/or diverticular disease.

Metabolic Disorders are for example selected from Obesity, Type 2 Diabetes Mellitus, Metabolic Syndrome, Non-Alcoholic Fatty Liver Disease (NAFLD), Hyperlipidemia (Dyslipidemia), and/or Insulin Resistance.

Autoimmune and Inflammatory Diseases are for example selected from Rheumatoid Arthritis, Systemic Lupus Erythematosus (SLE), Multiple Sclerosis (MS), Type 1 Diabetes Mellitus, Ankylosing Spondylitis, Psoriasis, Hashimoto's Thyroiditis and/or Sjögren's Syndrome.

Allergic and Atopic Conditions are for example selected from Asthma, Atopic Dermatitis (Eczema), Allergic Rhinitis (Hay Fever), Food Allergies and/or Eosinophilic Esophagitis. Neurological and Psychiatric Disorders are for example selected from autism spectrum disorders (ASD), Depression, Anxiety Disorders, Parkinson's Disease, Alzheimer's Disease, Schizophrenia, Bipolar Disorder and/or chronic fatigue syndrome/Myalgic Encephalomyelitis.

Cardiovascular Diseases are for example selected from Atherosclerosis, Hypertension (High Blood Pressure), coronary artery disease, and/or Heart Failure.

Skin Conditions are for example selected from Acne Vulgaris, Rosacea, Psoriasis, Atopic Dermatitis (Eczema), and/or Seborrheic Dermatitis.

Respiratory Conditions are for example selected from Chronic Obstructive Pulmonary Disease (COPD), Cystic Fibrosis, Asthma, and/or Allergic Rhinitis.

Cancers are for example selected from Colorectal Cancer, Gastric (Stomach) Cancer, Liver Cancer (Hepatocellular Carcinoma), Pancreatic Cancer, and/or Esophageal Cancer.

Genitourinary and Reproductive Health are for example selected from Bacterial Vaginosis, Urinary Tract Infections (UTIs), Preterm Birth and Preterm Labor, Infertility, Endometriosis, and/or prostate diseases.

Metabolic Bone Diseases are for example selected from Osteoporosis, Osteoarthritis, and/or Rheumatoid Arthritis.

Infectious Diseases are for example selected from Human Immunodeficiency Virus (HIV) Progression, Helicobacter pylori Infection, Clostridioides difficile Infection, and/or Viral Infections (e.g., Influenza).

Mental Health Disorders are for example selected from Depression, Anxiety, Stress-Related Disorders, and/or Post-Traumatic Stress Disorder (PTSD).

In another aspect, the present invention relates to a kit for use in a method according to the present description comprising:
a) at least one set of primers comprising forward primers targeting the 16S rRNA gene and reverse primers targeting the 5S rRNA gene wherein the sequence of the forward primer is SEQ ID NO: 3 and the sequence of the reverse primers is selected from SEQ ID NO: 1 and SEQ ID NO:2; and
b) instructions for use to detect the presence in a sample of polynucleotides sequences which encode the full-length sequence of a bacterial ribosomal RNA operon encompassing a 16S rRNA gene and a 5S rRNA gene.

### EXAMPLES

### Example 1: Method for identifying strain/sequence types of bacterial species and/or subspecies in a sample

The method comprises the following steps:

### DNA preparation

High molecular weight (HMW) genomic DNA was extracted from a biological sample using a standardized extraction protocol for long-read sequencing. The quality and concentration of the extracted DNA were assessed using a Qubit fluorometer (Thermo Fisher Scientific), ensuring that all samples met the minimum threshold for purity and concentration required for downstream applications.

### Primer Design

Ribosomal operon regions have been identified as the start (5'-region) of mature 16S rDNA gene (Condon et al., Microbial Rev. 59(4):623-45, 1995) and the end (3'-region) of the mature 5S rDNA gene.

For example, forward primers (16SFP) targeting the 16S rDNA gene are selected from SEQ ID NO: 3.
SEQ ID NO:3 is 5'-AGRRTTYGATYHTDGYTYAG-3' wherein
R is A or C;
Y is C or T/U;
H is A or C or T/U; and
D is A or G or T/U.

For example, reverse primers (5SRP) targeting the 5S rDNA gene are selected from SEQ ID NO: 1 and SEQ ID NO:2.
SEQ ID NO:1 is 5'- GTTCGGNATGGRWHSRGGYG-3' wherein
   R is A or C;
   N is a or c or g or t/u;
   W is a or t/u;
   H is a or c or t/u;
   S is c or g; and
   Y is c or t/u.
SEQ ID NO: 2 is 5'-GTTCGRNAWGGDDHSRSGYG-3' wherein
   R is A or C;
   N is A or C or G or T/U;
   W is A or T/U;
   H is A or C or T/U;
   S is C or G; and
   Y is C or T/U.

### Polymerase Chain Reaction

Ribosomal DNA was amplified using the primers 16SFP and 5SRP and the GoTaq^{®} Long PCR Master Mix (M4021, Promega, Madison, USA) on a thermocycler. In detail, amplification of targeted DNA shall be carried out in 25-µl reaction volumes, each containing 5ng of DNA, 12.5 µL of Master Mix (1x concentration), 0.5 µM of forward and reverse primers, respectively, and adjust the total volume to 25 µl by the addition of high-performance liquid chromatography-grade H2O.

The PCR was started with 1) 2min initial denaturation at 94°C, 2) 30s at 94°C, 3) 30s at 52°C (48°C-54°C) for annealing, 4) 5min at 65°C for elongation (2-10min) and 5) 10 min for final extension at 65°C. Steps 2-4 were repeated for 20-35 cycles.

### Library preparation

### a. End-prep

End-repair and A-tailing of the DNA fragments were performed using the NEBNext Ultra II End Prep module (New England Biolabs) as part of the Oxford Nanopore Native Barcoding Kit protocol (Oxford Nanopore, Oxford, UK).

In this step, 1 µg of fragmented DNA was subjected to a combined end-repair and A-tailing reaction in a single tube. The reaction was carried out according to the manufacturer's instructions. Briefly, the DNA was incubated with the NEBNext Ultra II End Prep reagents at 20°C for 5 minutes, followed by 65°C for 5 minutes. This process resulted in the repair of nicks, the generation of blunt ends, and the addition of a single adenine (A) nucleotide to the 3' ends of the DNA fragments, preparing them for subsequent adapter ligation.

Following adapter ligation, the DNA was purified using AMPure XP beads (Beckman Coulter) at a 1.8x bead-to-sample ratio. The purified DNA was then eluted in 10 µL of nuclease-free water. This step was essential to remove excess adapters, unligated DNA fragments, and any residual enzymes or reagents.

### b. Native Barcoding Ligation

Equimolar amounts of each barcoded sample were pooled to create a multiplexed library. The concentration of each individual barcoded sample was determined using a Qubit fluorometer, and the samples were pooled accordingly to ensure equal representation of each barcode in the final sequencing library.

### c. Adapter Ligation

The pooled library was subjected to a final ligation step where sequencing adapters compatible with the Oxford Nanopore Technologies sequencing platform were ligated to the DNA. This step was conducted using the ligation module provided in the Native Barcoding Kit 24 V14, according to the manufacturer's protocol.

### d. Final Purification and Quantification

The final library was purified using AMPure XP beads at a 1.0x bead-to-sample ratio to remove any unligated adapters and contaminants. The purified library was eluted in 15 µL of nuclease-free water and quantified using a Qubit fluorometer. The library was then diluted to the appropriate concentration for loading onto the Oxford Nanopore flow cell.

### Sequencing of the Ribosomal Operon DNA

The prepared library was loaded onto an Oxford Nanopore R10.4 flow cell according to the manufacturer's protocol. Sequencing was conducted using the appropriate ONT sequencing device, with run parameters set according to standard guidelines for multiplexed libraries.

This method enabled the simultaneous sequencing of up to 24 samples in a single run, with each sample uniquely identifiable by its respective barcode.

### Example 2: Annotation rrn-database creation

### An annotation rrn-database was created as follows:

The *rrn*-database of the present invention is composed of 50'000 (49'519) full-*rrn* sequences obtained from the high quality publicly available and complete genomes of the GTDB release 220 (Parks et al. 2018). The genomes were annotated with Prokka (1.14.0) (Seemann 2014) and *rrn* polynucleotide sequence copies extracted from the gff3 files with a python script.

The *rrn*-database is composed of polynucleotide sequences varying typically between 4800bp to 7500bp of nucleotides in length, with minor variants of 1700-3500bp of nucleotides in length wherein the 23S rRNA gene is missing.

Taxonomic resolution of the database was curated down to species and/or subspecies, i.e. strain level. The GTDB taxonomy nomenclature adopted in the *rrn*-database of the invention was completed down to nine ranks: Kingdom, Phylum, Class, Order, Family, Genus, Species, Strain and rRNA copy.

As an example, the information associated to the polynucleotide sequence in the database looks as follows:
d_Bacteria;p_Proteobacteria;c_Gammaproteobacteria;o_Enterobacterales;f_Ente robacteriaceae;g_Escherichia;s_Escherichia_coli;t_Escherichia_coli_ATCC-11775_RNA1.

The Strain rank was defined as "Genus_Species_Strain_RNA-copy", where Strain is filled with the isolate identifier indicated in the GTDB metatable or other identifier available, such as the sequence type.

For complete genomes with multiple *rrn* copies, the unique variants of *rrn* copies of the same genome were enumerated (e.g. RNA1, RNA2).

The database was completed with the reference *rrn* sequences of the genomes present in the ZymoBIOMICS Gut Microbiome Standard D6331.

The database will be regularly updated with new versions of GTDB database and additional *rrn* from complete bacterial or archaeal genomes.

The *rrn* database comprises reference sequences of bacterial ribosomal RNA operons and for each reference sequence, the taxonomic classification, the sequence type, and the serotype of the unique *rrn* copies per genome as an indication of the strain type of bacterial species and/or subspecies.

### Example 3: In silico Analysis of Strain-Level Taxonomic Annotation of five distinct Escherichia coli strains in the ZymoBiomics Gut Microbiome Standard D6331.

To evaluate the performance of accurate taxonomic annotation at the strain level for five distinct Escherichia coli strains within the ZymoBIOMICS Gut Microbiome Standard D6331, an in-silico analysis was conducted. This analysis involved the following steps:
Bacteria strains used in this study were obtained from the ZymoBIOMICS Gut Microbiome Standard D6331.

Genomes - Dataset Acquisition: The annotated reference genome sequences of all the strains present in the ZymoBIOMICS Gut Microbiome Standard D6331 were obtained from the manufacturer's website repository. The D6331 standard contains a defined mixture of microbial species, including five distinct *E*. *coli* strains, which is used as a benchmark for microbial community profiling based on two lengths of the ribosomal operon (*rrn*), once the full ribosomal operon (16S-ITS2-23S-ITS2-5S) of about 5.5Kbp and a shorter fragment of the *rrn* (16S-ITS1-23S) of about 4.5Kbp, respectively.

### Simulated Metagenomic Read Generation:

To simulate a realistic metagenomic dataset, the targeted regions of the full ribosomal operon (16S-ITS2-23S-ITS2-5S) of about 5.5Kbp and a smaller fragment of the *rrn* (16S-ITS1-23S) of about 4.5Kbp were extracted *in silico* from the reference genomes of all the strains present in the ZymoBIOMICS Gut Microbiome Standard D6331.

Thereafter, Oxford Nanopore reads with perfect quality scores were generated *in silico.* Copy number of the genomes were set to reflect the relative abundance indicated by the manufacturer normalized by the copy number of the *rrn* in the different genomes (see Table 1). Sequencing depth was adjusted to reflect realistic scenarios in microbiome studies. Two digital twin samples from ZymoBIOMICS Gut Microbiome Standard D6331 were generated, with full-*rrn* sequences (dD6331_16S-5S) and with 16S-23S (dD6331_16S-23S) sequences, respectively.

Bioinformatic Processing: the filtered reads were mapped to the GTDB-*rrn* with minimap2 (v2.24) (Li 2018). For each taxonomic classification, the read with the highest ratio between the DP Alignment Score (AS) and the Total number of mismatches and gaps in the alignment (NM) was selected as the representative sequence for that Amplicon Sequence Variance (ASV). The ASV-table was generated by counting the reads assigned to each taxonomic (annotation). A phyloseq object was first created with the ASV-table and ASVs of each sample and subsequently merged.

Results: This *in silico* analysis provides a comprehensive assessment of the capability to accurately resolve and annotate bacterial species and/or subspecies at the strain level in polymicrobial samples by means of the full length *rrn.* The accuracy of strain-level resolution was evaluated by comparing the annotated bacterial species, and particularly the five different *E. coli* strains in the digital Mock samples (dD6331_16S-5S) and dD6331_16S-23S) as compared with the theoretical Mock composition (tD6331_adjusted) included in the ZymoBIOMICS Gut Microbiome Standard D6331 as declared by the manufacturer's instructions in the Genomic DNA column (see Table 1 below).

**Table 1, part 1**

| | **tD6331** | **tD6331 adjusted** | **dD63311 6S-23S (4.5kbp)** | **dD6331 16S-5S (5.5kbp)** |
|---|---|---|---|---|
| Bacteroides_fragilis_Zymo-OBEAV111D6FAA | 14 | 14.42 | 11.44 | 10.73 |
| Faecalibacterium_prausnitzii_Zym o-AP34BHI | 14 | 14.42 | 16.91 | 17.44 |
| Roseburia_hominis_Zymo-OBEAV111DCM | 14 | 14.42 | 8.54 | 8.01 |
| Veillonella_rogosae Zymo-AC2811ANNA2 | 14 | 14.42 | 18.26 | 17.12 |
| Bifidobacterium_adolescentis_Zy mo-LMG10502 | 6 | 6.18 | 5.03 | 4.72 |
| Fusobacterium_nucleaturn_Zymo-2150A | 6 | 6.18 | 8.62 | 8.08 |
| Limosilactobacillus_fermentum_Zym o-B1840 | 6 | 6.18 | 11.08 | 10.39 |
| Prevotella_corporis_Zymo-OB21FMU4 | 6 | 6.18 | 5.73 | 5.38 |
| Escherichia_coli_Zymo-B1109 | 2.8 | 2.88 | 1.52 | 2.04 |
| Escherichia_coli_Zymo-B2207 | 2.8 | 2.88 | 0.38 | 1.41 |
| Escherichia_coli_Zymo-B3008 | 2.8 | 2.88 | 2.69 | 2.63 |
| Escherichia_coli_Zymo-B766 | 2.8 | 2.88 | 0.22 | 1.35 |
| Escherichia_coli_Zymo-JM109 | 2.8 | 2.88 | 0.00 | 1.37 |
| Akkermansia_muciniphila_Zymo-OB21FAANB28 | 1.5 | 1.54 | 1.48 | 1.74 |
| Clostridioides_difficile_Zymo-P4D3A11 | 1.5 | 1.54 | 3.02 | 2.84 |
| Methanobrevibacter smithii | 0.1 | 0.10 | 0.00 | 0.00 |
| Salmonella enterica | 0.01 | 0.01 | 0.00 | 0.00 |
| Enterococcus faecalis | 0.001 | 0.001 | 0.00 | 0.00 |
| Clostridium perfringens | 0.0001 | 0.0001 | 0.00 | 0.00 |
| Bifidobacterium_adolescentis_ATCC -15703 | 0 | 0 | 5.08 | 4.76 |
| Candida albicans - Fungi | 1.5 | 0 | Fungi not tested | Fungi not tested |
| Saccharomyces cerevisiae - Fungi | 1.5 | 0 | Fungi not tested | Fungi not tested |
| Total relative abundance (%) | 100.00 | 100.00 | 100.00 | 100.00 |

All five *E. coli* strains in the ZymoBIOMICS Gut Microbiome Standard D6331 could be detected by targeting the full length *rrn* of 5.5kbp including ITS2 and 5S rDNA gene (see Table 1, column dD6331_16S-5S), which is a significant improvement in strain detection and annotation accuracy as compared to the sequencing of only the fragment from 16S-23S of 4.5kbp (see column dD6331_16S-23S). The ribosomal operon region limited to 16S-23S failed to detect one out of five strains (*E. coli* JM109) and highly underestimated the relative abundance of two out of five strains (*E. coli* B2207 and *E. coli* B766).

The present analysis has shown that different haplotypes of ribosomal operon copies can exist within the same genome. The different haplotypes can vary in the presence or absence of entire genes, like the complete lack of the 23S rDNA gene or the variation of the ITS1 region between the 16S and 23S ribosomal subunit genes including (i) no tRNA or (ii) any combination of tRNA.

The majority or the *rrn* have a length between 4.6-6.0kbp. Some minor *rrn* variants lacking the 23S rDNA gene with a length of 1.7-2kbp have been observed in the species *Roseburia hominis.* The species containing short haplotypes of the *rrn* would remain undetected or underestimated by targeting the 16S-23S target sequencing.

## Claims

1. A method for identifying strain types of bacterial species and/or subspecies in a sample the method comprising the steps of:
a) obtaining a sample containing one or more strain types of bacterial species and/or subspecies;
b) extracting DNA from said sample;
c) detecting from the extracted DNA the presence of at least one or more polynucleotides sequences which encode the full-length sequence of a bacterial ribosomal RNA operon encompassing a 16S rRNA gene and a 5S rRNA gene;
d) analyzing said at least one or more polynucleotides sequences as predictive of the identity of strain types of bacterial species and/or subspecies and comparing the at least one or more polynucleotides sequences with reference sequences of bacterial ribosomal RNA operon in a *rrn*-database; and
e) identifying the strain types of bacterial species and/or subspecies based on the at least one or more polynucleotides sequences which encode sequences of bacterial ribosomal RNA operons detected in the sample.

2. The method of claim 1, wherein the bacterial ribosomal RNA operon encompasses from 5' to 3':
a) a 16S rRNA gene;
b) a non-coding regions ITS1;
c) one or more tRNA gene;
d) a 23S rRNA gene;
e) a non-coding regions ITS2; and
f) one or more tRNA gene; and
g) a 5S rRNA gene,
or the bacterial ribosomal RNA operon encompasses from 5' to 3':
a) a 16S rRNA gene;
b) a non-coding regions ITS;
d) one or more tRNA gene; and
e) a 5S rRNA gene.

3. The method of any one of claims 1-2, wherein the *rrn*-database comprises reference sequences of bacterial ribosomal RNA operons and for each reference sequence, the taxonomic classification, the sequence type, and the serotype of the unique *rrn* copies per genome as an indication of the strain type of bacterial species and/or subspecies.

4. The method of any one of the preceding claims, wherein the detecting step c) is carried out by a polymerase chain reaction using at least one set of primers for amplifying the full-length sequence of a bacterial ribosomal RNA operon encompassing a 16S rRNA gene and a 5S rRNA gene.

5. The method of claim 4, wherein the at least one set of primers comprises forward primers targeting the 16S rRNA gene and reverse primers targeting the 5S rRNA gene.

6. The method of claim 5, wherein the sequence of the reverse primers is selected from the group comprising SEQ ID NO: 1, SEQ ID NO: 2 and/or the sequence of the forward primer is SEQ ID NO: 3.

7. The method of any one of the preceding claims, further comprising a step of preparing a DNA library comprising the at least one or more polynucleotides sequences detected after step c).

8. The method of any one of the preceding claims, wherein the analyzing step d) is carried out by DNA sequencing analysis of said at least one or more polynucleotides sequences.

9. The method of any one of the preceding claims, wherein the sample containing one or more strain types of bacterial species and/or subspecies is obtained from a subject.

10. The method of any one of claims 1-9, wherein said sample is selected from the group consisting of whole blood, serum, saliva, sputum, urine, cerebrospinal fluid, stool, amniotic fluid, tissue sample such as biopsy, synovial fluid, swab sample, or a sample containing liquid or solid colonies of bacteria.

11. The method of any one of claims 9-10, further comprising a step of treating the subject with at least one antibacterial agent against the strain types of bacterial species and/or subspecies identified after step e).

12. The method of any one of claims 9-11, wherein the subject is a human patient having a disease or health condition caused by or related to bacterial infection, including nosocomial infection, otitis, conjunctivitis, pneumonia, bacteremia, sinusitis, pleural empyema and endocarditis, intravascular or endothelial infections, osteomyelitis, meningitis and chronic respiratory diseases such as asthma, chronic obstructive pulmonary disease (COPD), occupational lung diseases and pulmonary hypertension.

13. The method of any one of claims 9-12, wherein the subject is a human patient having a disease or health condition caused by or related to the microbiome or microbiota and selected from the group comprising gastrointestinal disorders, metabolic disorders, autoimmune and inflammatory diseases, allergic and atopic conditions, neurological and psychiatric disorders, cardiovascular diseases, skin conditions, respiratory conditions, cancer, genitourinary and reproductive health diseases, metabolic bone diseases, infectious diseases, mental health disorders, chronic kidney disease, non-alcoholic steatohepatitis (NASH), fibromyalgia, chronic fatigue syndrome and/ or allergic diseases.

14. The method of any one of claims 1-8, wherein the sample is selected from the group consisting of food, food supplement, water, soil, air or filtered samples.

15. A kit for use in a method according to any one of claims 1-14 comprising
a) at least one set of primers comprising forward primers targeting the 16S rRNA gene and reverse primers targeting the 5S rRNA gene wherein the sequence of the forward primer is SEQ ID NO: 3 and the sequence of the reverse primers is selected from SEQ ID NO: 1 and SEQ ID NO:2;
b) instructions for use to detect the presence in a sample of polynucleotides sequences which encode the full-length sequence of a bacterial ribosomal RNA operon encompassing a 16S rRNA gene and a 5S rRNA gene.
